# EUROPEAN PATENT SPECIFICATION

(11) **EP 4 353 145 B1**
(45) Date of publication and mention of the grant of the patent: **09.04.2025**
(21) Application number: 23020472.9
(22) Date of filing: 13.10.2023
(51) Int. Cl.: A61B 5/00, A61B 5/22

(54) **DOLORIMETRIC ADAPTER FOR A HAND GRIP DYNAMOMETER**
DOLORIMETERADAPTER FÜR HANDGRIFF-DYNAMOMETER
ADAPTATEUR DOLORIMÈTRIQUE POUR DYNAMOMÈTRE À POIGNÉE

(30) Priority: 14.10.2022 PL 44252922
(43) Date of publication of application: 17.04.2024
(73) Proprietor: Uniwersytet Mikolaja Kopernika w Toruniu, 87-100 Torun (PL)
(72) Inventor: Smuczynski, Wojciech, 85-791 Bydgoszcz (PL); Napierala, Waldemar, 85-794 Bydgoszcz (PL); Smuczynski, Andrzej, 85-791 Bydgoszcz (PL); Radziminska, Agnieszka, 86-065 Lochowo (PL); Weber-Rajek, Magdalena, 85-733 Bydgoszcz (PL); Nowacka, Agnieszka, 85-150 Bydgoszcz (PL); Sniegocki, Maciej, 85-435 Bydgoszcz (PL)

(56) References cited:
- EP-B1- 2 482 716
- JP-A- 2007 267 766
- US-A- 3 672 219
- US-A1- 2011 144 541
- US-A1- 2019 159 714
- US-A1- 2022 202 361
- YOUSAF SAAD N ET AL: "Human-Robot Interaction: Muscle Activation and Angular Location Affect Soft Tissue Stiffness", 2022 9TH IEEE RAS/EMBS INTERNATIONAL CONFERENCE FOR BIOMEDICAL ROBOTICS AND BIOMECHATRONICS (BIOROB), IEEE, 21 August 2022 (2022-08-21), pages 1 - 6, XP034219364, DOI: 10.1109/BIOROB52689.2022.9925462

## Description

The subject of the invention is dolorimetric adapter to hand grip dynamometer designed for pain objectification of patients during a palpation.

So far, dolorimeters, that is medical devices for measuring the intensity of pain during pressure, have been known from the industry. Such devices consist of a movable part, which is used to apply pressure to certain points on the body, and a meter connected to it, allowing to determine the strength of the pressure released.

A dolorimetric device is known from US5012817A which contains an element with a property which changes depending on the pressure exerted on it. A device is provided for attaching the segment to the user's hand so that the segment fits under the user's finger. The segment is sized to fit between the finger and the object touched by the user, while allowing essential tactile communication with the finger. A device for quantifying the variance of said property from said element is provided.

A system for measuring sensitivity is known from WO2019204899A1 to be used in patient's diagnostics and treatment, which includes: a handheld, formable device that contains a pressure sensor, a Bluetooth^{®} radio and a battery, the pressure sensor and Bluetooth radio in electrical communication with the battery, the pressure sensor in electrical communication with the Bluetooth radio; a computer device, a computer device that contains a Bluetooth^{®} receiver that is in radio communication with the Bluetooth radio, a processor and memory, the memory containing instructions for calibrating the system for a specific patient for calibration, the memory storing a set of calibration and sensitivity data for a specific patient; and a user interface that communicates electronically with the computer device.

A computer operated sensory testing system is known from EP2482716A2, which can be used for further studies on pain and supporting clinical diagnosis and treatment of pain syndromes. The system includes actuators to deliver pressure/strain (tension), auditory, olfactory and other stimuli to the patient. The system includes software to control the delivery of the stimuli. The system is further capable of receiving feedback on the stimuli received.

The purpose of the invention is to develop such a dolorimetric adapter that, when used together with a hand grip dynamometer, will allow an objective study of pain sensation in both inpatient and outpatient settings. The ease of use of the indicated invention will make it possible to accurately determine points of maximum soreness before conducting therapy and to compare the results after therapeutic intervention. Obtaining accurate, objective measurements significantly improves the quality of the treatment performed.

The invention allows for increased testing capability with a single device, in this case a hand grip dynamometer, without the need to purchase and calibrate further measuring devices. The use of a hand grip dynamometer for testing gives the possibility of acting with greater force, up to 120 kg.

The body of the adapter is placed on the handle of the dynamometer, after which a cap is placed in its upper part to lock the body. Inside the cap, there is a pressure plate, which is further tightened using a thumb screw. A pressure pin is screwed into the lower part of the body, along with a cap that directly exerts pressure on the patient's body. Making the overlays in two sizes allows testing with a similar pressure area for the index finger (16mm diameter) and for the thumb (20mm diameter).

The solution is an additional device, dedicated for use with a hand grip dynamometer. The indicated solution allows to increase the possibility of testing with one device, without the need to purchase and calibrate further measuring devices. The use of a hand grip dynamometer for testing gives the possibility of acting with a much greater force, up to 120 kg, compared to commercially available dolorimeters.

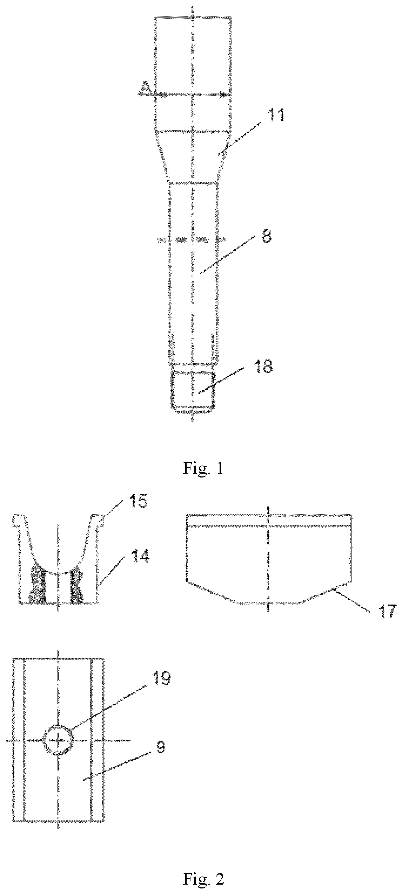

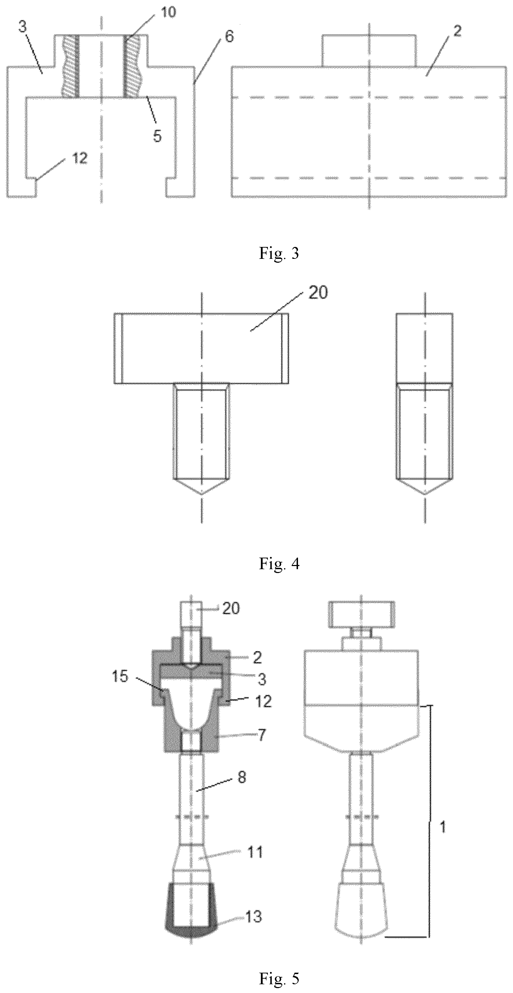

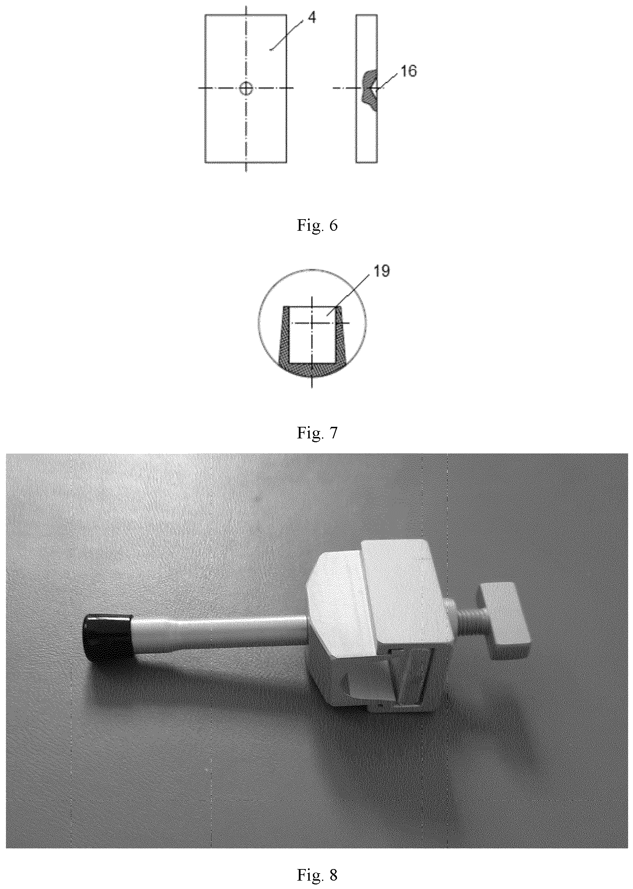

The object of the invention is visualized in the drawing, in which fig. 1 shows the pressure pin, fig. 2 the bottom cap fig. 3 the sleeve fig. 4 the screw fig. 5 the dolorimetric adapter, fig. 6 the pressure plate, fig. 7 a blind hole in the bottom cap, and fig. 8 the dolorimetric adapter with hand grip dynamometer.

The invention has been presented with examples of construction.

Example I. The dolorimetric adapter for the hand grip dynamometer consists of a body 1 embedded in a sleeve 2. Sleeve 2 has the shape of a channel, with side walls 6. The side walls 6 have the same dimension and are parallel to each other. Sleeve 2 has a base 3 and two side walls 6, set to the base 3 at right angles. The side walls 6 are L-shaped and their block serifs 12 face each other.

On the lower base 3 of the sleeve 2 there is a cuboid pressure plate 4 matching the dimensions of the upper wall 5 of the base 3 of the sleeve 2.

Sleeve 2 has a through-hole 10 for a screw 20 preferably wing-head thumb screw, pressing the sleeve 2 against the body 1 through the pressure plate 4. The pressure plate 4 has a blind seating hole 16, placed in one axis with the through-hole 10.

The body 1 consists of a channel-shaped bottom cap 7 and a pressure pin 8. In the upper wall 9 of the bottom cap 7, there is a blind hole 19 in which the pressure pin 8 seated through a threaded connection 18 is embedded. The pressure pin 8 is cylindrical in shape, and has an expansion 11 in the upper part, on which the locking cap 13 is seated.

The bottom cap 7 has side walls 14. The side walls 14 have the same dimensions and are parallel to each other. The side walls 14 have block serifs 15 facing in opposite directions to each other.

Block serifs 15 of the bottom cap 7 are adjusted in length and width to the internal dimensions of block serifs 12 of the sleeve 2.

The top wall 9 of the bottom cap 7 has two symmetrical bevels relative to the vertical axis of the bottom cap 7.

Example II. Dolorymetric adapter to hand grip dynamometer is characterized in that it consists of a body 1 embedded in sleeve 2 on pressure plate 4. The channel-shaped sleeve 2 has mutually parallel side walls 6 and a base 3 embedded at a right angle, which side walls 6 are L-shaped and have their block serifs 12 facing each other, and the sleeve 2 having a through hole 10 with a screw 20. On the bottom base 3 of sleeve 2, there is a pressure plate 4. The body 1 consists of a channel-shaped bottom cap 7 and pressure pin 8, with the upper wall 9 of bottom cap 7 having a blind hole 19 in which a cylindrical pressure pin 8 with an expansion 11 in the upper part is embedded. The bottom cap 7 has mutually parallel side walls 14 with block serifs 15 facing in opposite directions.

Example III. Example III is different from I due to having a cuboid-shaped pressure plate 4 with dimensions not greater than the width and length of the upper wall 5 base 3 of sleeve 2 embedded on the bottom base 3 of sleeve 2.

### Legend

1. Body
2. Sleeve
3. Base
4. Pressure plate
5. Sleeve's upper wall
6. Sleeve's side wall
7. Bottom cap
8. Pressure pin
9. Bottom cap's upper wall
10. Hole
11. Pressure pin's expansion
12. Block serif of sleeve's side wall
13. Safety cap
14. Bottom cap's side wall
15. Block serifs of bottom cap's side wall
16. Seating hole
17. Symmetrical bevels
18. Threaded connection
19. Blind hole
20. Screw

## Claims

1. Dolorimetric adapter for a hand grip dynamometer is **characterized in that** it consists of a body (1) seated in a channel-shaped sleeve (2) on a pressure plate (4), where the channel-shaped sleeve (2) has side walls (6) parallel to each other and a base (3) embedded at right angles to each other, with the side walls (6) being L-shaped and their block serifs (12) facing each other, and the sleeve (2) having a through hole (10) with a screw (20), on whose sleeve's (2) bottom base (3) a pressure plate (4) is embedded and body (1) consists of a channel-shaped bottom cap (7) and a pressure pin (8), where in the upper wall (9) of the bottom cap (7) there is a blind hole (19) where a cylindrical pressure pin (8) with an expansion (11) in its upper part is embedded, whereas the bottom cap (7) has mutually parallel side walls (14) with block serifs (15) pointing in opposite directions.

2. The adapter according to claim 1 is **characterized in that** the side walls (6) have the same dimension.

3. The adapter according to claims 1 or 2, is **characterized in that** the pressure plate (4) is rectangular in shape, preferably matching the dimensions of the upper wall (5) of the base (3) of the sleeve (2) or has dimensions not greater than the width and length of the upper wall (5) of the base (3) of the sleeve (2).

4. The adapter according to claims 1, 2 or 3 is **characterized in that** the screw (20) is a wing-head thumb screw.

5. The adapter according to claims 1, 2, 3 or 4, is **characterized in that** the pressure pin (8) has thread (18) in its lower part.

6. The adapter according to claims 1, 2, 3, 4 or 5, is **characterized in that** in its upper part the pressure pin (8) has a safety cap (13).

7. The adapter according to claims 1,2,3,4,5 or 6 is **characterized in that** the side walls (14) have the same dimension.

8. The adapter according to claims 1,2,3,4,5,6 or 7, is **characterized in that** the block serifs (15) of the bottom cap (7) are matched in length and width to the inner dimensions of the block serifs (12) of the sleeve (2).

9. The adapter according to claims 1,2,3,4,5,6,7 or 8 is **characterized in that** the upper wall (9) of the bottom cap (7) has two symmetrical bevels (17) with respect to the vertical axis of the bottom cap (7).

10. The adapter according to claims 1,2,3,4,5,6,7,8 or 9 is **characterized in that** the pressure plate (4) has a blind seating hole (16) placed in one axis with a through hole (10).

## Patentansprüche

1. Dolorimeteradapter für Handgriff-dynamometer ist **dadurch gekennzeichnet, dass** er aus einem Körper (1) besteht, der in einer Kappe (2) in Form eines Kanals auf der Druckplatte (4) montiert ist, wobei die Kappe (2) die Form eines Kanals hat und parallele Seitenwände (6) sowie eine Basis (3) aufweist, die im rechten Winkel angebracht ist, die Seitenwände (6) haben eine L-Form und sind mit Balken-Serifs (12) zueinander gerichtet, zudem besitzt die Kappe (2) eine Durchgangsbohrung (10) mit einer Schraube (20), auf deren auf unterer Basis (3) der Kappe (2) die Druckplatte (4) montiert ist, und der Körper (1) besteht aus einer Bodenkappe (7) in Form eines Kanals und einer Druckspindel(8), wo sich in der oberen Wand (9) der Bodenkappe (7) eine Sacklochbohrung (19) befindet, in der die zylindrische Druckspindel (8) mit einer Verlängerung (11) im oberen Teil eingesetzt ist, und die Bodenkappe (7) hat parallele Seitenwände (14) mit Balken-Serifs (15), die in entgegengesetzte Richtungen weisen.

2. Adapter nach Anspruch 1, **dadurch gekennzeichnet, dass** die Seitenwände (6) die gleichen Abmessungen aufweisen.

3. Adapter nach Anspruch 1 oder 2, **dadurch gekennzeichnet, dass** die Druckplatte (4) die Form eines Quaders hat und vorzugsweise in ihren Abmessungen an die obere Fläche (5) der Basis (3) der Kappe (2) angepasst ist oder Abmessungen aufweist, die nicht größer sind als die Breite und Länge der oberen Fläche (5) der Basis (3) der Kappe (2).

4. Adapter nach Anspruch 1, 2 oder 3, **dadurch gekennzeichnet, dass** die Schraube (20) eine Flügelschraube ist.

5. Adapter nach Anspruch 1, 2, 3 oder 4, **dadurch gekennzeichnet, dass** die Druckspindel (8) im unteren Bereich ein Gewinde (18) aufweist.

6. Adapter nach Anspruch 1, 2, 3, 4 oder 5, **dadurch gekennzeichnet, dass** die Druckspindel (8) im oberen Bereich eine Schutzkappe (13) besitzt.

7. Adapter nach Anspruch 1, 2, 3, 4, 5 oder 6, **dadurch gekennzeichnet, dass** die Seitenwände (14) die gleichen Abmessungen aufweisen.

8. Adapter nach Anspruch 1, 2, 3, 4, 5, 6 oder 7, **dadurch gekennzeichnet, dass** die Balken-Serifs (15) der Bodenkappe (7) in Länge und Breite an die Innenmaße der Balken-Serifs (12) der Kappe (2) angepasst sind.

9. Adapter nach Anspruch 1, 2, 3, 4, 5, 6, 7 oder 8, **dadurch gekennzeichnet, dass** die obere Wand (9) der Bodenkappe (7) zwei symmetrische Abschrägungen (17) bezüglich der vertikalen Achse der Bodenkappe (7) aufweist.

10. Adapter nach Anspruch 1, 2, 3, 4, 5, 6, 7, 8 oder 9, **dadurch gekennzeichnet, dass** die Druckplatte (4) eine Sackbohrung (16) aufweist, die mit der Durchgangsbohrung (10) fluchtet.

## Revendications

1. Adaptateur dolorimétrique pour dynamomètre à poignée **caractérisé en ce qu'**il se compose d'un corps (1) monté dans un manchon (2) sous forme de profilé en U sur une plaque de pression (4), où le manchon (2) sous forme de profilé en U a des parois latérales parallèles (6) et une base (3) montée à angle droit et ces parois latérales (6) sont en forme de L et se font face avec les empattements de poutre (12), et la manchon (2) a un trou traversant (10) avec une vis (20), sur cette base inférieure (3) du manchon (2) est montée la plaque de pression (4), et le corps (1) se compose d'une plaquette inférieure (7) sous forme de profilé en U et d'une goupille de pression (8), où dans la paroi supérieure (9) de la plaquette inférieure (7) se trouve un trou borgne (19) où est monté la goupille de pression (8) sous forme de cylindre avec extension (11) dans la partie supérieure, et la plaquette inférieure (7) a des parois latérales parallèles (14) avec les empattements de poutre (15) dirigés dans les directions opposées.

2. Adaptateur selon la revendication 1, **caractérisé en ce que** les parois latérales (6) ont la même dimension.

3. Adaptatuer selon la revendication 1 ou 2, **caractérisé en ce que** la plaque de pression (4) a la forme d'un parallélépipède, de préférence ses dimensions sont adaptées à la paroi supérieure (5) de la base (3) du manchon (2) ou ses dimensions ne sont pas supérieures à la largeur et à la longueur de la paroi supérieure (5) de la base (3) du manchon (2).

4. Adaptateur selon la revendication 1, 2 ou 3, **caractérisé en ce que** la vis (20) est une vis papillon.

5. Adaptateur selon la revendication 1, 2, 3 ou 4, **caractérisé en ce que** la goupille de pression (8) a un filetage (18) dans sa partie inférieure.

6. Adaptateur selon la revendication 1, 2, 3, 4 ou 5, **caractérisé en ce que** dans la partie supérieure la goupille de pression (8) a une plaquette de protection (13).

7. Adaptateur selon la revendication 1, 2, 3, 4, 5 ou 6, **caractérisé en ce que** les parois latérales (14) ont la même dimension.

8. Adaptateur selon la revendication 1, 2, 3, 4, 5, 6 ou 7, **caractérisé en ce que** les empattements de poutre (15) de la plaquette inférieure (7) sont adaptés en longueur et en largeur aux dimensions intérieures des empattements de poutre (12) du manchon (2).

9. Adaptateur selon la revendication 1, 2, 3, 4, 5, 6, 7 ou 8, **caractérisé en ce que** la paroi supérieure (9) de la plaquette inférieure (7) a deux découpes symétriques (17) par rapport à l'axe vertical de la plaquette inférieure (7).

10. Adaptateur selon la revendication 1, 2, 3, 4, 5, 6, 7, 8 ou 9, **caractérisé en ce que** la plaque de pression (4) a un trou de montage borgne (16) aligné avec le trou traversant (10).
